# EUROPEAN PATENT APPLICATION

(11) **EP 3 557 252 A1**
(43) Date of publication of application: **23.10.2019**
(21) Application number: 17881045.3
(22) Date of filing: 04.12.2017
(51) Int. Cl.: G01N 33/52, A61J 1/05, G01N 33/48

(54) **INSPECTION CARTRIDGE AND BLOOD INSPECTION DEVICE**

(30) Priority: 14.12.2016 JP 2016242116
(71) Applicant: Konica Minolta, Inc., Tokyo 100-7015 (JP)
(72) Inventor: MAJIMA Masanao, Tokyo 100-7015 (JP); OTANI Makiko, Tokyo 100-7015 (JP); MATHUO Masataka, Tokyo 100-7015 (JP); MURAYAMA Takanori, Tokyo 100-7015 (JP)
(74) Representative: Hoffmann Eitle
(86) International application number: PCT/JP2017/043471
(87) International publication number: WO 2018/110344

(57) **Abstract**

To provide an inspection cartridge (10) capable of preventing a user from using a blood sample of a wrong vacuum blood collection tube when the user dispenses the blood sample from the vacuum blood collection tube to the inspection cartridge (10), and a blood inspection device (20) which performs a blood inspection using the same. An inspection cartridge (10) used for performing a blood inspection with a blood inspection device (20) provided with an identifier (12) for identifying an anticoagulant usable in the inspection cartridge (10).

## Description

### Technical Field

The present invention relates to an inspection cartridge used when inspecting blood collected at the time of blood inspection by a blood inspection device and a blood inspection device which performs a blood inspection by using the same, and more specifically relates to an inspection cartridge and a blood inspection device capable of easily visually recognizing information of a vacuum blood collection tube which may be used in the inspection and of reducing inspection errors.

### Background Art

In recent years, a blood inspection device capable of measuring one or a plurality of items is used in a blood inspection.

In the blood inspection using the blood inspection device, a blood sample is collected from a subject using a vacuum blood collection tube or the like, and the blood sample is dispensed into an inspection cartridge. By mounting the inspection cartridge on the blood inspection device, the blood inspection is executed.

In a case where whole blood is used in the blood inspection, an anticoagulant is added to the vacuum blood collection tube used when collecting the blood sample from the subject such that the whole blood is not coagulated.

For example, heparin, sodium citrate, ethylenediaminetetraacetic acid (EDTA), sodium fluoride and the like are used as the anticoagulant, and the anticoagulant which may be used is different according to inspection items to be measured.

In a case where the blood inspection is performed using the whole blood to which a wrong anticoagulant is added, it is impossible to obtain a correct inspection result, and in a case where this is overlooked and a wrong inspection result is reported to a user such as a doctor and a nurse, there is a fear that wrong treatment is performed, so that it is important to check the anticoagulant included in the vacuum blood collection tube when collecting the blood.

Therefore, a type of the anticoagulant to be added is clearly described on the vacuum blood collection tube and a cap of the vacuum blood collection tube is made a predetermined color such that wrong selection of the vacuum blood collection tube is prevented.

In JIS T 3233: 2011, recommended character codes and color codes are designated according to the type of the anticoagulant added to the vacuum blood collection tube as illustrated in Table 1.

**[Table 1]**

| ADDITIVE | | CHARACTER CODE | COLOR CODE |
|---|---|---|---|
| EDTA | DIPOTASSIUM SALT | K2E | LAVENDER |
| | TRIPOTASSIUM SALT | K3E | LAVENDER |
| | DISODIUM SALT | N2E | LAVENDER |
| TRISODIUM CITRATE | 9:1 | 9NC | LIGHT BLUE |
| TRISODIUM CITRATE | 4:1 | 4NC | BLACK |
| FLUORIDE/OXALATE | | FX | GRAY |
| FLUORIDE/EDTA | | FE | GRAY |
| FLUORIDE/HEPARIN | | FH | GREEN |
| HEPARIN LITHIUM | | LH | GREEN |
| HEPARIN SODIUM | | NH | GREEN |
| CITRATE, PHOSPHATE, GLUCOSE, ADENINE | | CPDA | YELLOW |
| NONE | | Z | RED |

The user such as the doctor, the nurse, and a clinical laboratory technician selects a vacuum blood collection tube which may be used in the inspection out of such vacuum blood collection tubes, and performs the blood inspection as described above. Note that the type of the vacuum blood collection tube which may be used in the inspection is, for example, confirmed on a list written on paper and the like, or designated and displayed by a clinical inspection information system (LIS) or the like.

However, there is a case where the blood sample is dispensed from the different type of vacuum blood collection tube into the inspection cartridge to perform the blood inspection by human errors such as mistaking or misunderstanding by the user and by work without checking the list due to familiarity.

Patent Literature 1 discloses a method of discriminating at the time of blood inspection whether the anticoagulant used in the collected blood sample is the wrong anticoagulant on the basis of monovalent ion concentration, divalent cation concentration and the like, and reporting the fact in a case where this is the blood sample collected using the wrong anticoagulant.

### Citation List

### Patent Literature

Patent Literature 1: JP 2011-508223 A

### Summary of Invention

### Technical Problem

However, the method in Patent Literature 1 is for preventing a wrong inspection result from being reported to a user by the blood sample collected using the wrong anticoagulant. For this reason, it is known after the blood inspection whether or not this is the blood sample collected using the wrong anticoagulant, so that a useless blood inspection is performed.

For this reason, it is desired that the user be aware of this before dispensing the blood sample from the wrong vacuum blood collection tube to the inspection cartridge and performing the blood inspection.

In addition, such blood inspection is conventionally carried out using an automated blood inspection device by a clinical laboratory technician who specializes in inspection in an inspection room, but in recent years, need for point of care testing (POCT) to quickly inspect beside the subject such as at bed side and quickly obtain results is increasing.

In such inspections, not only experts in inspection such as the clinical laboratory technicians but also doctors and nurses sometimes carry out the inspection beside the conventional medical work, so that a system capable of performing the inspection with reduced risk of the erroneous inspection more easily is required.

In view of such a situation, an object of the present invention is to provide an inspection cartridge capable of preventing a user from using a blood sample of a wrong vacuum blood collection tube and a blood inspection device which performs a blood inspection using the same when the user dispenses the blood sample from the vacuum blood collection tube to the inspection cartridge.

### Solution to Problem

The present invention is achieved to solve the problem in the conventional technology as described above, and in order to realize at least one of the above-described objects,
an inspection cartridge reflecting an aspect of the present invention is an inspection cartridge used for performing a blood inspection with a blood inspection device,
the inspection cartridge provided with an identifier for identifying an anticoagulant usable in the inspection cartridge.

Also, a blood inspection device reflecting an aspect of the present invention is
a blood inspection device which performs a blood inspection by using the above-described inspection cartridge, provided with
an input means for inputting information included in the identifier of the inspection cartridge.

### Advantageous Effects of Invention

According to the present invention, since information of an anticoagulant which may be used in an inspection item and the like is clearly indicated on an inspection cartridge or a packaging package thereof, it is possible to urge a user to pay attention when the user dispenses a blood sample from a vacuum blood collection tube to the inspection cartridge, and it is possible to prevent the use of the blood sample from a wrong vacuum blood collection tube.

Also, according to the present invention, it is possible to confirm in advance a type of the anticoagulant included in the vacuum blood collection tube and a type of the anticoagulant which may be used for the inspection cartridge, and collect the inspections of the blood samples collected by using the vacuum blood collection tubes including the same anticoagulant, so that it is possible to improve efficiency of the blood inspection.

Furthermore, according to the present invention, in the blood inspection device, the type of the anticoagulant included in the vacuum blood collection tube is compared with the type of anticoagulant which may be used by the inspection cartridge, and if they do not coincide with each other, a warning is issued in advance or the blood inspection is not performed, so that useless blood inspection may be prevented.

### Brief Description of Drawings

Fig. 1 is a schematic diagram illustrating a schematic configuration of an inspection cartridge in one aspect of the present invention.
Fig. 2 illustrates a display example of an identifier.
Fig. 3 is a schematic configuration diagram for illustrating a blood inspection device for performing a blood inspection using the inspection cartridge in Fig. 1.
Fig. 4 is a schematic diagram illustrating a schematic configuration of an inspection cartridge in another aspect of the present invention.

### Description of Embodiments

Hereinafter, an embodiment (example) of the present invention is described in further detail with reference to the drawings.

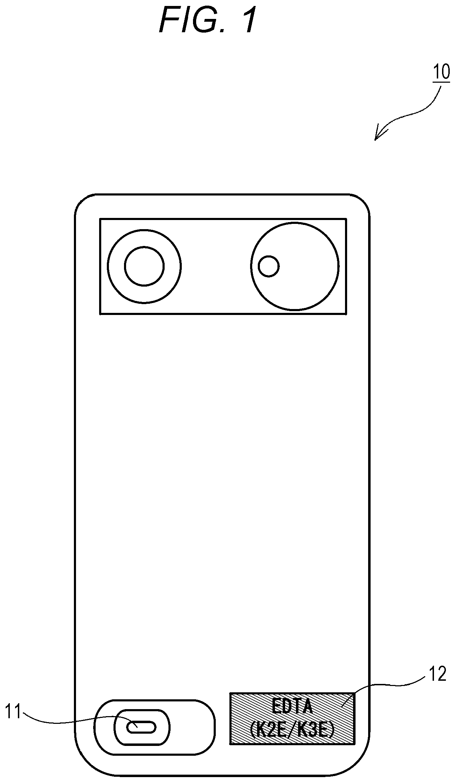

Fig. 1 is a schematic diagram illustrating a schematic configuration of an inspection cartridge in an aspect of the present invention.

As illustrated in Fig. 1, an inspection cartridge 10 of this example is provided with an identifier 12 for identifying an anticoagulant which may be used.

Note that, the identifier 12 may be provided anywhere in a position not interfering with a function of the inspection cartridge 10; however, in order to improve visibility of a user, this is preferably provided in the vicinity of a specimen dispensing port 11 of the inspection cartridge 10.

Also, as the identifier 12, a bar code or a radio frequency identifier (RFID) including a character such as a name of the anticoagulant and a character code, a color corresponding to a color code defined for each anticoagulant, information regarding the anticoagulant and the like may be used, or a combination thereof may be used.

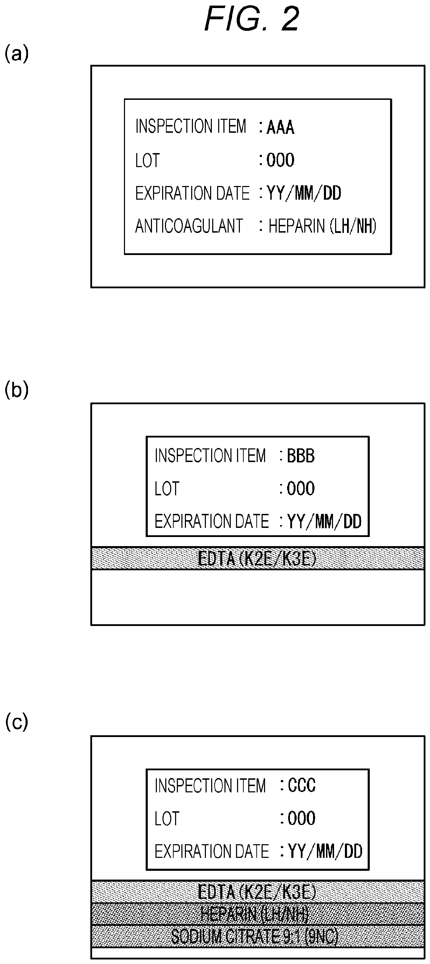

Fig. 2 illustrates a display example of the identifier 12. As illustrated in Fig. 2(a), the information regarding the anticoagulant may be displayed only with characters, or as illustrated in Fig. 2(b), the information regarding the anticoagulant may be displayed by characters and a color.

Also, in a case of the inspection cartridge 10 capable of supporting a plurality of anticoagulants, as illustrated in Fig. 2(c), the identifier 12 including the information regarding a plurality of anticoagulants may be provided.

Also, as illustrated in Fig. 2, not only the information regarding the anticoagulant but also information regarding the inspection cartridge such as inspection items performed using the inspection cartridge 10, a lot number of the inspection cartridge 10, an expiration date, and manufacturer information may also be included in the identifier 12.

Note that the display example of the identifier 12 illustrated in Fig. 2 is merely an example, and any display is available as long as the information regarding the anticoagulant is displayed so as to be easily visually recognized.

Note that the character code of the anticoagulant is a code set for each anticoagulant; for example, a character code recommended in JIS T 3233: 2011 as illustrated in Table 1 above may be used, or an individually set character code may be used.

As the color code, for example, a color code recommended in JIS T 3233: 2011 as illustrated in Table 1 above may be used, or an individually set color code may be used. Note that in a case of individually setting the color code, it is preferable to set the color code matching with a color of a cap of a vacuum blood collection tube used by the user.

Note that the bar code is not especially limited as long as this is a bar code decodable by a computer or the like, and this may be not only a one-dimensional code but also a two-dimensional code.

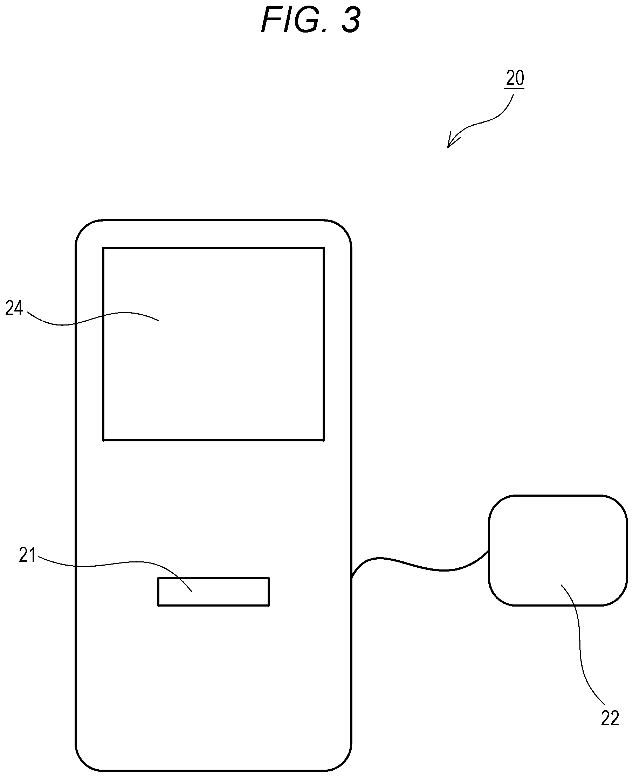

As illustrated in Fig. 3, the inspection cartridge 10 of this example configured in this manner may perform a blood inspection as follows using a blood inspection device 20 provided with an input means 22.

Note that the input means 22 may be appropriately changed according to the identifier 12 of the inspection cartridge 10; for example, in a case where the identifier 12 is the character or color, the keyboard, mouse, touch panel and the like may be used, and in a case where the identifier 12 is the barcode, RFID and the like, an identifier reading means such as a barcode reader and an RFID reader may be used.

In a case where the identifier reading means is used as the input means 22, it is preferably configured such that the information regarding the anticoagulant included in the identifier 12 is displayed on a display means 24 of the blood inspection device 20 by reading the identifier of the inspection cartridge 10 by the identifier reading means.

Note that the identifier reading means may be an external device connected to the blood inspection device 20 as illustrated in Fig. 3, but this may also be an internal device provided inside the blood inspection device 20. In a case where the identifier reading means is the internal device, it is possible to configure to read the identifier 12 of the inspection cartridge 10 in a state in which the inspection cartridge 10 is mounted on a cartridge mounting unit 21 of the blood inspection device 20.

Also, the blood inspection device 20 is preferably configured to compare the information of the anticoagulant included in the vacuum blood collection tube used for the inspection with the information of a usable anticoagulant included in the identifier 12 input by the input means 22, and to issue a warning in advance by displaying the warning on the display means 24 or not to execute the blood inspection when they do not coincide with each other.

Note that the information of the anticoagulant included in the vacuum blood collection tube to be used may be input by the user using the input means 22. Also, in a case where the vacuum blood collection tube is provided with the bar code or RFID including the information of the anticoagulant, it is possible to read the information of the anticoagulant included in the vacuum blood collection tube by using the identifier reading means as the input means 22.

It is also possible to configure such that the blood inspection device 20 obtains the information of the anticoagulant included in the vacuum blood collection tube from a clinical inspection information system (LIS) by connecting the clinical inspection information system (LIS) to the blood inspection device 20.

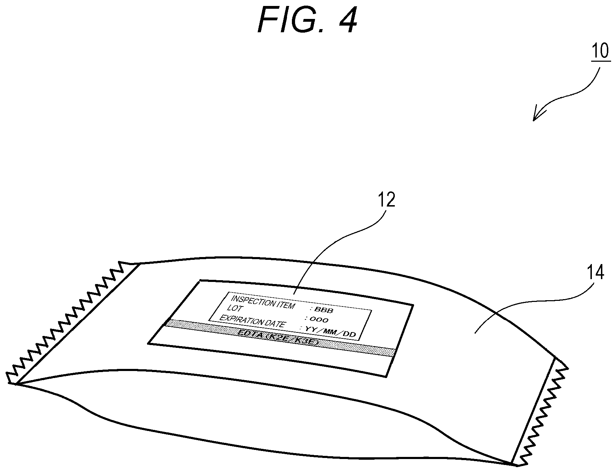

Fig. 4 is a schematic diagram for illustrating a schematic configuration of an inspection cartridge in another aspect of the present invention.

In this example, the same components as those of the inspection cartridge 10 illustrated in Figs. 1 and 2 are assigned with the same reference numerals, and the detailed description thereof is omitted.

In this example, an inspection cartridge main body is enclosed in a packaging package 14. By thus enclosing the inspection cartridge main body in the packaging package 14, it is possible to protect the inspection cartridge main body from an external environment, and it is possible to prevent deterioration of the inspection cartridge main body.

In the inspection cartridge 10 configured in this manner, the above-described identifier 12 is provided on the packaging package 14.

Note that the identifier 12 may be provided on both the packaging package 14 and the inspection cartridge main body.

Although the preferred aspects of the present invention are described above, the present invention is not limited thereto, and various modifications may be made without departing from the object of the present invention.

### Reference Signs List

- 10: Inspection cartridge
- 11: Specimen dispensing port
- 12: Identifier
- 14: Packaging Package
- 20: Blood inspection device
- 21: Cartridge mounting unit
- 22: Input means
- 24: Display means

## Claims

1. An inspection cartridge used for performing a blood inspection with a blood inspection device,
the inspection cartridge provided with an identifier for identifying an anticoagulant usable in the inspection cartridge.

2. The inspection cartridge according to claim 1,
wherein the identifier is provided in the vicinity of a specimen dispensing port of the inspection cartridge.

3. The inspection cartridge according to claim 1, comprising:
an inspection cartridge main body; and
a packaging package which encloses the inspection cartridge main body,
wherein the identifier is provided on the packaging package.

4. The inspection cartridge according to claim 3,
wherein the identifier is provided on the inspection cartridge main body.

5. The inspection cartridge according to any one of claims 1 to 4,
wherein the identifier is at least any one of:
at least any character of a name of the anticoagulant and a character code;
a color according to a color code defined for each anticoagulant; and
at least any one of a barcode and a radio frequency identifier (RFID) including information regarding the anticoagulant.

6. The inspection cartridge according to claim 5,
wherein the color code is a color code recommended in JIS T 3233: 2011.

7. The inspection cartridge according to claim 5,
wherein the color code is a color code matching with a color of a cap of a vacuum blood collection tube used by a user.

8. The inspection cartridge according to any one of claims 1 to 7,
wherein the identifier includes information regarding a plurality of anticoagulants.

9. The inspection cartridge according to any one of claims 1 to 8,
wherein the identifier includes information regarding the inspection cartridge.

10. A blood inspection device which performs a blood inspection by using the inspection cartridge according to any one of claims 1 to 9, the device comprising:
an input means for inputting information included in the identifier of the inspection cartridge.

11. The blood inspection device according to claim 10, further comprising:
a display means for displaying information included in the identifier input by the input means.

12. The blood inspection device according to claim 10 or 11,
wherein the blood inspection device compares information of an anticoagulant included in a vacuum blood collection tube used for an inspection with information of a usable anticoagulant included in the identifier input by the input means, and issues a warning when the pieces of information do not coincide with each other.

13. The blood inspection device according to any one of claims 10 to 12,
wherein the blood inspection device compares the information of the anticoagulant included in the vacuum blood collection tube used for the inspection with the information of the usable anticoagulant included in the identifier input by the input means, and does not execute the blood inspection when the pieces of information do not coincide with each other.

14. The blood inspection device according to claim 12 or 13,
wherein the information of the anticoagulant included in the vacuum blood collection tube used for the inspection is input by using the input means.

15. The blood inspection device according to claim 14,
wherein at least any one of a barcode and a radio frequency identifier (RFID) including the information of the anticoagulant is input by using the input means.

16. The blood inspection device according to claim 12 or 13,
wherein the blood inspection device is connected to a clinical inspection information system, and
the blood inspection device obtains the information of the anticoagulant included in the vacuum blood collection tube used for the inspection from the clinical inspection information system.
